# EUROPEAN PATENT SPECIFICATION

(11) **EP 1 927 849 B1**
(45) Date of publication and mention of the grant of the patent: **25.04.2018**
(21) Application number: 07291411.2
(22) Date of filing: 27.11.2007
(51) Int. Cl.: G01N 27/07, G01N 33/18

(54) **Conductivity measurement device**
Vorrichtung zur Messung der Leitfähigkeit
Dispositif de mesure de conductivité

(30) Priority: 01.12.2006 FR 0655276
(43) Date of publication of application: 04.06.2008
(73) Proprietor: EMD Millipore Corporation, Burlington, MA 01803 (US)
(72) Inventor: DIMITRAKOPOULOS, Aristotelis, F-28130 Saint Martin De Nigelles (FR); RAJAGOPALAN, Pascal, 93600 Aulnay-Sous-Bois (FR); LE NINIVIN, Cèline, 78480 Verneuil Sur Seine (FR); GAIGNET, Yves, 78180 Montigny Le Bretonneux (FR); BEJTLICH III, Chester L., US-Reading, MA 1867 (US)
(74) Representative: Santarelli

(56) References cited:
- EP-A- 1 598 664
- JP-A- 5 322 832
- US-A- 4 626 413
- US-A- 5 275 957
- US-B1- 6 444 474
- US-B1- 6 734 021
- DANIEL D., GUTZ, I.G.R.: "Microfluidic cells with interdigitated array gold electrodes: Fabrication and electrochemical characterization" TALANTA, vol. 68, 2005, pages 429-436, XP002440361
- Alex Wekhof: "Treatment of Contaminated Water, Air and Soil With UV Flashlamps", Environmental Progress, vol. 10, no. 4, 1 November 1991 (1991-11-01), pages 241-247, XP055050363,

## Description

The present invention concerns the manufacturing of devices for measuring the conductivity of an ultrapure liquid, such as ultrapure water, in particular for devices for measuring organic substances or Total Organic Carbon (TOC) in a sample of liquid.

Numerous modern technological applications require ultrapure water for their operation, in particular in the chemical, pharmaceutical, medical and electronic industries.

Currently, as is described for example in the patent US 4 767 995, the conductivity measurement cells used for example in water purification systems, are composed of at least two parts of electrode-forming conductive material mounted head to foot on the same element body of insulating material with axial overlap. At least one of the electrodes is hollow so as to receive within its hollow the other electrode coaxially with the first part. The space between the two electrodes defines a sample volume on which the measurement is performed.

Such an arrangement is adapted to enable a sufficiently low cell constant to be obtained to enable the measurement of the conductivity of an ultrapure liquid.

It is to be recalled, in this respect, that conductivity is the measurement of the flow of electrons which pass through a substance. It is directly proportional to the concentration of ions, to the charge carried by each of those ions (valency) and to their mobility. This mobility depends on the temperature, and consequently, the measurement of the conductivity also depends on the temperature.

In theoretically pure water, the only two ionic species present arise from the dissociation of water molecules into H⁺ and OH⁻.

Thus, at 25°, the theoretical conductivity of a water sample free from ionic contaminant is equal to 0.055 µS/cm, i.e. a resistivity (inverse of the conductivity) of 18.2 MQ.cm.

This conductivity is measured by applying an electric potential between the two electrodes immersed in the water sample. It is determined from the voltage and the strength of the current produced within the conductivity measurement cell.

This conductivity measurement is affected by the geometry of the cell, the total surface area of the electrode (s) and the distance separating them (L).

These last two parameters define the cell constant: cell constant = L/s.

In practice, the greater the surface area of the electrode, the higher the strength of the current generated for a given voltage and thus the more precise the current measurement. This means that the lower the cell constant, the more precise the measurement.

This is particularly important in the case of ultrapure water. This is because low cell constants (< 0.2 cm⁻¹ in practice) are necessary to obtain a high signal that will be less subject to interference.

One of the favored applications for this type of conductivity measurement cell is the measurement of the Total Organic Carbon (TOC), as described for example in the patent application EP 0 498 888 or the patent US 6 741 084. In practice, a sample of theoretically ultrapure water is subjected to photo-oxidation by means of ultraviolet rays (UV) of which the wavelength is approximately 185 nm, which makes it possible to measure the quantity of organic carbon in the water on the basis of the drop in resistivity resulting from the oxidation by ultraviolet of the organic substances present in the sample of water subjected to the measurement.

Currently, in the mass production method, the elements constituting those cells are generally assembled by hand, which results in particular in variations in the geometry of the cells with respect to the specifications or from one item thereof to another, such as a variation in the relative position of the two electrodes. In practice, this results in variations in the cell constant which affect the precision of the conductivity measurement.

US 6,444,474 discloses a device for measuring the total organic carbon content of a sample of liquid, as defined in the preamble of claim 1.

In general terms, the present invention is directed to arrangements making it possible to manufacture devices for measuring conductivity of an ultrapure liquid with very high precision and furthermore leading to other advantages.

It provides, more particularly, a device for measuring the total organic carbon content of a sample of liquid comprising a device for measuring conductivity of a liquid, in particular ultrapure water, comprising two conductivity measurement electrodes suitable for defining a cell constant enabling the measurement of the conductivity of an ultrapure liquid, each electrode taking the form of a pattern of electrically conductive material on a substrate of electrically insulating material, a cell having a conductivity measurement chamber communicating with the outside of the cell by a liquid inlet and outlet provided in the cell, and at least partially covering the patterns of the conductivity measurement electrodes, and at least one window transparent to ultraviolet rays to perform photo-oxidation of the sample of liquid located in the measurement chamber, characterized in that it comprises for the photo-oxidation, a xenon flash lamp emitting ultraviolet rays of a wavelength greater than or equal to 160 nm and less than or equal to 400 nm, and in that it is adapted to perform a conductivity measurement between two flashes of the xenon flash lamp.

Thus, the present invention not only makes it possible to ensure manufacture with very high precision, in particular from the point of view of the thickness of the electrodes, and thus minimum tolerances, but also to eliminate the manual assembly of cell components during their manufacture by using an automated manufacturing technique, in particular considerably increasing the reproducibility of the cell constant from one cell to another.

In practice, conductivity measurement cells in accordance with the invention are manufactured using manufacturing technologies arising from electronics, such as microlithography or screen printing. They are advantageously manufactured by photoetching of electrode patterns on a given substrate material, such as a polymer, for example Mylar® (polyester), or a ceramic, such as quartz glass, it also being possible for the electrode-forming material which is deposited to vary. This is preferably carbon, boron-doped diamond, platinum, silver, gold or titanium.

A temperature sensor, in practice taking the form of a thermistor, is advantageously provided to be placed either upstream or downstream of the conductivity measurement cell or, better still, positioned under one of the electrodes with, possibly, a fine glass interface between the temperature sensor and the space receiving the sample to measure.

This is because one of the major problems in the field of conductivity measurement is that this is greatly affected by variations in temperature. More particularly, the higher the temperature of a sample, the lower the resistivity (due to the mobility of the ions). Thus, to ensure a precise measurement, it is necessary to temperature-compensate the conductivity measurement. To that end, conductivity measurement cells are generally equipped with sample temperature sensors.

Preferably, the temperature sensor is formed using the same manufacturing techniques as those mentioned above for manufacturing the electrodes. Its pattern may thus be formed on the substrate, which makes it possible to precisely position the sensor with respect to the conductivity measurement electrodes and to avoid the damage which may result from the assembly methods of the state of the art. It may be made from polysilicon or boron-doped diamond , for example. In both cases, the substrate is then chosen of quartz glass due to the high temperatures which are required for the deposit. By virtue of these arrangements, it is possible to produce an integrated sensor making it possible to determine the temperature of the sample of the liquid analyzed at the same time as its conductivity, thus eliminating the measurement errors of the conductivity due to the temperature measurement errors.

Furthermore, by virtue of the present invention, a cell window placed on the substrate can be manufactured with the same precision as the substrate, and consequently the sample volume will be reproducible and much smaller than the sample volume necessary with the current technical solutions. This leads to a shorter conductivity measurement time (direct measurement) for the ultrapure water or for any other ultrapure liquid to measure.

The two parts may be assembled using a seal. However, by virtue of the present invention, the two parts, i.e. the substrate and the cell window, may be manufactured with planar surfaces and only a small amount of pressure is then sufficient to retain the two parts against each other in order to maintain fluid-tightness. Thus, there will be no need to bond the two parts together, nor to use a fluid-tight seal.

The absence of adhesive makes it possible in particular to eliminate the organic contaminants coming therefrom and leading to errors in measurement in the case of measuring TOC.

Moreover, the present invention makes it possible to optimize the TOC measurement chamber in terms of volume and thickness of the liquid layer in order for them to be sufficiently small to ensure effective photo-oxidation by UV. Furthermore, the arrangement according to the invention advantageously lends itself to a further development in which the photo-oxidation process is improved, by using a catalytic layer.

A photocatalyst based on a semiconductor material with a wide energy band may be used, preferably based on titanium oxide according to the teachings described in the following documents:
1-Advanced Photochemical Processes, EPA/625/R-98/004, December 1998
2-Photocatalytic oxidation of Gas-Phase BTEX-contaminated Waste Streams, NREL/TP-473-7575, March 1995
3-Photocatalytic thin film cascade for treatment of organic compounds in wastewater, A.H.C. Cha,, J.P. Barford, C.K. Cha,, Water Science and Technology, vol 44, 5, 187-195

Titanium dioxide, more particularly in its anatase form, is the preferred catalyst, since it is the most effective in photo-catalytic reactions.

However, other semiconductors such as ZnO, Fe₂O₃, CdS, ZnS, SrTiO₃, CaTiO₃, KTaO₃, Ta₂O₅, and ZrO₂ may be use (M.R. Hoffmann, S.T. Martin, W. Choi., D.W. Bahnemann, Chem. Rev. 95, 69, 1995 ; A. Fujishima, T.N. Rao, D.A. Tryk, *J. Photochem. Photobiol. C : Photochemistry.,* 1, 1, 2000).

It may also be envisaged to produce mixtures of different forms of photo-catalyst, for example the rutile and anatase forms for titanium dioxide. Furthermore, M. Penpolchaoren, R. Amal, M. Brungs, *Journal of Nanoparticle Research*, 3, 289, 2001 describe the photo-degradation of saccharose and nitrates by virtue of titanium dioxide particles covered with nano-hematites (TiO₂/Fe₂O₃).

To improve the kinetics of the reaction, transition metals or doping ions are preferably introduced, such as iron, silver or platinum on particles of titanium oxide. The efficiency of TiO₂ suspension modified by the silver ion has thus been studied in the context of the mineralization of saccharose (V. Vamathevan, R. Amal, D. Beydoun, G. Low, S. McEvoy, *J. Photochem. Photobiol.A : Chemistry.,* 148, 233, 2002). The authors have shown the improvement in the reduction of the oxygen by virtue of a better electrons-holes separation with Ag/TiO₂ modified particles compared with particles of pure TiO₂. Thus it has been possible to improve the mineralization speed of certain selective organic compounds (H. Tran, K. Chiang, J. Scott, R. Amal, *Photochem. Photobiol. Sci.,* 4, 565, 2005). It will also be noted that the photo-decomposition of phenol has been studied with a photocatalyst based on TiO₂/SiO₂, which may also be implemented in the context of the present invention.

In practice, as regards titanium dioxide for example, this may be implemented in the form of particles, a deposit on the electrodes (in whole or in part) and/or the substrate, but also in the form of a film of titanium dioxide which may cover the interior portion of the container in contact with the sample.

Also in practice, the wide energy band of the semiconductor material of the photocatalyst has a value less than or equal to that of the titanium dioxide, i.e. of the order of 3.2 eV.

The TOC measurement device uses a xenon flash type UV lamp emitting a wavelength in the spectrum ranging from UV at 160 nm to the visible spectrum at 400 nm.

Such a lamp, which may also be used in association with a photocatalyst of the aforementioned type, has the particular characteristic of providing intense power pulses of short duration.

Thus, according to one embodiment, a small volume for the sample of ultrapure liquid (less than 300 µl in practice) is provided in a conductivity measurement cell manufactured with the techniques arising from microelectronics and comprising an integrated temperature sensor, titanium oxide deposited on the substrate as a photocatalyst and a UV lamp taking the form of a light-emitting diode of which the wavelength is 365 nm (the use of a light-emitting diode is not claimed).

The photocatalyst may totally or partially cover one or each conductivity measurement electrode pattern or else not be in contact with such a pattern.

Other electronic components relative to the measurement device, such as an analog to digital converter, microcontrollers, a power supply for the UV light emitting diode lamp, etc. are furthermore advantageously integrated into the device, by printing them for example on the back of the substrate, so as to miniaturize the entire device. Thus, a miniature TOC analyzer may be produced capable of being connected to an item of equipment as a complete sub-assembly.

Advantageously, an optical sensor is arranged on the same side as the back of the substrate to detect the signal of the UV light emitting diode for the purpose of quantifying and adjusting the real quantity of energy passing through the ultrapure liquid sample present in the TOC analyzer. Such an arrangement enables real-time detection of the completion of the photo-oxidation step on the basis of the intensity of the signal and also makes it possible to detect possible irregularities within the conductivity measurement cell, such as air bubbles or particles liable to absorb and/or deviate the signal.

The completion of the photo-oxidation step may thus be detected in a simpler and more certain manner than by the methods currently used, i.e., either the determination of the slope of the first and/or second derivative of the curve of conductivity against time, or stopping the photo-oxidation at a given time.

The measurements thus made may also be used to differentiate and quantify the different types of organic material present in the ultrapure liquid sample analyzed, the case arising using a filter to quantify the full spectrum or a specific wavelength (for example 365 nm).

According to preferred arrangements relative to that measuring device, which may possibly be combined:
- the cell comprises two complementary members, one comprising a recess forming the conductivity measurement chamber and the other forming the substrate, the liquid inlet and outlet of the cell being formed in the substrate-forming member,
- the device comprises a casing in two parts by virtue of which the cell is housed and means for assembly by clamping the two parts suitable for ensuring the fluid-tightness of the conductivity measurement cell,
- the electrically insulating material of the substrate is chosen from the group comprising quartz glass, Mylar® and silicium,
- the conductivity measurement electrode-forming electrically conductive material is chosen from the group comprising carbon, platinum, silver, gold, titanium and boron-doped diamond,
- the conductivity measurement electrode patterns take the form of two interleaved combs forming an interdigitated structure,
- the device further consists of producing a thermistor on the substrate by forming a pattern thereon with conductor or semiconductor material,
- the material of the thermistor is chosen from the group comprising polysilicon, platinum and boron-doped diamond,
- the or each conductivity measurement electrode-forming pattern, when produced on the underlying layer of thermistor-forming material, is separated therefrom by a deposit of electrically insulating material, preferably silicon dioxide SiO₂ or silicon nitride Si₃N₄,
- the device comprises electronic components specific to the device, on the rear face of the substrate,
- the device comprises a photo-catalyst deposit based on semiconductor material with a wide energy band on the substrate,
- the wide energy band semiconductor material comprises at least one of a single oxide of a transition metal, a mixed oxide of a transition metal and of an alkali or alkaline-earth metal and of a transition metal sulfide, preferably doped,
- the semiconductor material is chosen from the group comprising Ti0₂, ZnO, Fe₂O₃, ZrO₂, Ta₂O₅, SrTiO₃, CaTiO₃, KTaO₃, CdS and ZnS.
- it comprises an optical sensor arranged at the rear face of the substrate to detect the ultraviolet rays emitted by the ultraviolet lamp,
- the electrode patterns are formed by etching a layer of material deposited beforehand on the substrate or on an underlying layer itself deposited on that substrate.

Other advantages of the present invention will appear from the reading of the following description, made with respect to the drawings in which:
- Figure 1 is an exploded perspective view of a device for measuring conductivity of an ultrapure liquid, in accordance with a preferred embodiment of the present invention;
- Figure 2 is a longitudinal median cross-section, at a larger scale, of the device of Figure 1;
- Figure 3 is a very diagrammatic plan view, representing the patterns for the conductivity measurement electrodes and the thermistor-forming pattern of the measuring device of Figures 1 and 2; and
- Figure 4 is a longitudinal median cross-section view of a TOC measuring device.

It should be noted in this connection that the description which follows is that of a preferred embodiment, given by way of non-limiting example.

The device 1 for measuring conductivity of an ultrapure liquid, here ultrapure water, comprises a casing 10 in two parts, 10, 11 of plastics material, here machined, by virtue of which there are housed a window 12 transparent to UV, here of quartz glass, and a substrate 13, also of quartz glass and having several motifs. This window 12 is provided with a recess 14 forming a conductivity measurement chamber adapted to receive the liquid sample to analyze.

With the substrate 13 it forms the actual conductivity measurement cell.

The substrate 13 comprises, to that end, two deposits of electrically conductive material forming patterns of electrodes 15, 16 for conductivity measurement and another deposit of semiconductor material forming a thermistor 17 which serves to determine the temperature of the liquid sample present in the chamber 14. These deposits are made on the face of the substrate 13 that faces the conductivity measurement chamber 14 and they terminate with soldering surfaces 18 situated at one of the ends of the substrate 13, for the connection of electrical connection wires 19 of an electrical connector 20 making it possible to electrically connect the conductivity measurement cell 12, 13 to one or more exterior circuits for determining the temperature and the temperature-compensated conductivity of the sample of ultrapure water present in the chamber 14. The electrode patterns 15, 16 are of course made so as to obtain a low cell constant, in practice less than 0.2 cm⁻¹, enabling the measurement of the conductivity of ultrapure water. In fact, the strips of electrically conductive material forming electrodes are of such dimensions and arrangement as to obtain that cell constant. Furthermore, the substrate 13 comprises two water supply holes 21, 22 which respectively correspond to two holes 23, 24 made in the lower part 11 of the casing, for the purpose of allowing the inlet and outlet of the water analyzed in the chamber 14, in a direction orthogonal to that of the circulation of water therein. Two 'O' ring seals 25, 26 each received in a hollow 27, 28 formed in the bottom of the lower part 11 serve to provide fluid-tightness with the substrate 13 posed on top. On the other hand, there is no seal between the substrate 13 and the window 14, fluid-tightness being achieved solely by the clamping of the upper part 10 of the casing onto the lower part 11 using four screws 29a-29d cooperating with internal screw threads 30a-30d formed in the lower part 11 of the casing.

It will moreover be noted that the upper part 10 of the casing is provided with a central opening 31 enabling optical access to the chamber 14, while the lower part 11 has a cavity 32 opening between the two holes 23, 24 formed in the lower part 11 of the casing and arranged so as to be situated under the conductivity measurement chamber 14 and, thereby, the opening 31 formed in the upper part 10 of the casing.

It will also be noted that those lower 11 and upper 10 parts are machined so as to form projections 33, 34 suitable for holding the conductivity measurement cell 12, 13 in place between the lower 11 and upper 10 parts, once the screws 29a-29d have been tightened.

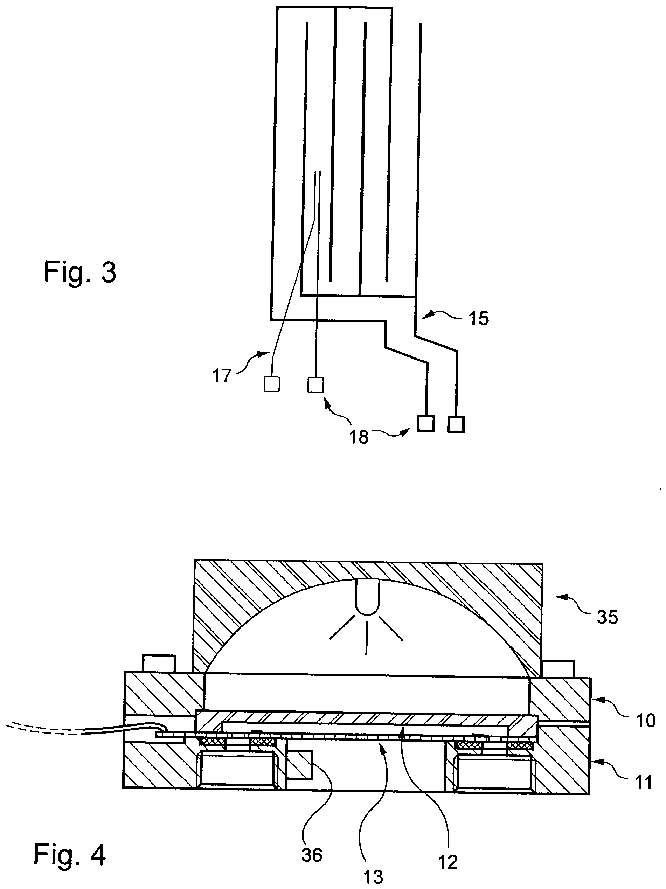

As can be best seen in Figure 3, the two conductivity measurement electrodes 15, 16 take the form of an interdigitated structure formed by two interleaved combs, whereas the thermistor 17 is formed by two strips of semiconductor material connected to each other at the opposite end to that which terminates with the soldering surfaces 18.

In practice, these deposits are, for example, carried out by the implementation of a microelectronics method comprising in particular the following steps:
- depositing polysilicon on the substrate 13 of quartz glass;
- forming the pattern of the temperature sensor 17 in the layer of polysilicon, preferably after P-type doping and activation;
- depositing electrically insulating material, here silicon nitride Si₃N₄, on at least a portion of the polysilicon pattern;
- depositing titanium oxide;
- forming the patterns of the conductivity measurement electrodes 15, 16 in the titanium;
- depositing titanium oxide as a photo-catalyst;
- metallization or on chromium (Cr/Au) and forming the connection areas 18.

In practice, LPCVD (Low Pressure Chemical Vapor Deposition) methods may be used, in particular for the deposit of Si₃N₄ and polysilicon. The patterns are formed by dry attack or attack in a liquid medium.

One of the favored applications of such a conductivity measuring device is its use for the measurement of the Total Organic Carbon (TOC) in a sample of liquid.

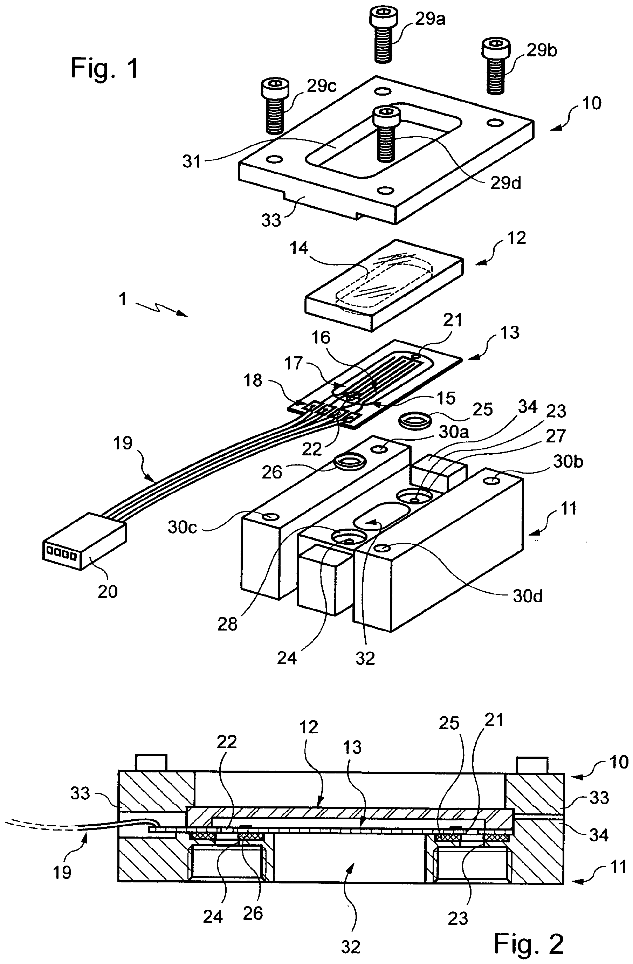

For this, the device described with the aide of Figures 1 to 3 is completed, as can be seen in Figure 4, with an ultraviolet (UV) lamp 35, here by virtue of a light-emitting diode (the use of a light-emitting diode is not claimed), mounted on the upper surface of the upper part 10 of the casing so as to be able to irradiate the conductivity measurement chamber 14 through the opening 31 formed in that upper part.

Moreover, the cavity 32 formed in the lower part 11 is advantageously exploited for the installation of an optical sensor 36 making it possible to detect the signal of the UV lamp 35 for the purpose of quantifying and adjusting the real quantity of energy passing through the sample of ultrapure liquid present in the measurement chamber 14.

Advantageously too, the part of the rear face of the substrate overhanging that cavity may be utilized for the installation of other electronic components related to the TOC measuring device, as indicated above.

The devices for measuring conductivity of an ultrapure liquid and the TOC measurement/verification devices implementing them have, by virtue of the present invention, the following advantages:
1. complete and miniaturized TOC measurement device that is capable of being connected to an item of equipment as a complete sub-assembly
2. elimination of manual assembly on manufacture of these devices
3. effective photo-oxidation
4. increased life of the ultraviolet generating devices
5. possibility of adding a stage for control of the UV
6. better control and adjustment of the emission of the UV by electronics
7. high reproducibility of the UV emission from one TOC measuring device to another
8. possibility of detecting air bubbles, particles, etc. within the conductivity measurement cell
9. reduction of the noise linked to electrical interference, which makes it possible to measure the conductivity at the same time as the photo-oxidation process occurs, and, therefore, possibility of controlling the photo-oxidation process until its completion
10. high reproducibility of the cell constant, and, therefore, necessity to calibrate only a small percentage of a given batch of cells
11. high reproducibility of the temperature sensors in a given batch.
12. obtainment of better temperature detection precision during the conductivity measurements
13. reduction in the manufacturing costs
14. reduction in the photo-oxidation time due to the fact that it is possible to implement a smaller sample volume than those of the state of the art
15. direct measurement due to that small sample volume to measure
16. flexibility in the design of the cells
17. flexibility of incorporation of the conductivity measurement cell in a given device (system for water purification, reservoir, etc.) while respecting the detection limit provided (values in ppt in practice for TOC measuring devices)
18. single use of the conductivity cell may be envisaged
19. elimination of organic contaminants in the absence of the use of adhesive
20. possibility of miniaturizing the TOC measuring device with the possibility of integrating therein various additional electronic components and devices for measurement or detection
21. possibility of obtaining a low cell constant suitable for measuring the conductivity of an ultrapure liquid while reducing the volume of the conductivity measurement chamber to a minimum

It is also to be noted that the xenon UV flash lamp as claimed, advantageously coupled to the optical sensor, makes it possible to dissipate the photo-thermal heat supplied by the lamp between the flashes. This has the advantage of not heating the sample. Chemical interference is thus limited. It is known in this connection that in the presence of silica heating of the sample of ultra pure water will lead to disturbance of the conductivity (in particular a reduction).

This absence of heating also limits the contribution of error and uncertainty linked to a high compensation for temperature by an algorithm.

According to one operating mode, the TOC measuring device is implemented with continuous recording of the conductivity, which makes it possible to view the end of the oxidation and to have a dynamic response from oxidation.

In terms of performance, this TOC measuring device makes it possible to take conductivity measurements in real time (in practice, 1 point /s) by accessing the characteristic conductivity profiles of the organic compounds, with possibilities for organic detection at the scale of the ppb, or even smaller.

This detection limit of this device to the ppb is, as a matter of fact, in particular enabled by virtue of recording data approximately every second.

Recording by the second is advantageously enabled by the use of a xenon UV flash lamp, since, between two flashes, a measurement may be coupled and thus no photonic interference perturbs the measurement. No filtering is thus necessary to obtain access to a precise measurement of the temperature and the conductivity.

The fact of performing a measurement every second also makes it possible to limit chemical interferences due to possible dissolving of CO₂ in the sample of water, due, for example, to defective fluid-tightness.

The fact of performing a measurement every second also makes it possible to limit chemical interferences due to possible dissolving CO₂ in the sample of water, due, for example, to defective fluid-tightness.

By virtue of these recorded conductivity profiles, this device may furthermore be adapted to differentiate the behavior of ions, for example from carbon dioxide (CO₂), and of contaminants, for example dichloromethane (CH₂ Cl₂).

More generally, the present invention is not limited to the embodiments described or represented, but covers any variant form.

Other electrodes patterns may in particular be envisaged, such as two straight parallel electrodes or two interleaved electrodes, one C-shaped and the other straight.

## Claims

1. A device for measuring the total organic carbon content of a sample of liquid comprising a device for measuring conductivity of a liquid, in particular ultrapure water, comprising two conductivity measurement electrodes (15, 16) suitable for defining a cell constant enabling the measurement of the conductivity of an ultrapure liquid, each electrode taking the form of a pattern of electrically conductive material on a substrate (13) of electrically insulating material, a cell having a conductivity measurement chamber (14) communicating with the outside of the cell by a liquid inlet and outlet (21, 22) provided in the cell, and at least partially covering the patterns of the conductivity measurement electrodes, and at least one window (12) transparent to ultraviolet rays to perform photo-oxidation of the sample of liquid located in the measurement chamber (14), **characterized in that** it comprises, for the photo-oxidation, a xenon flash lamp emitting ultraviolet rays of a wavelength greater than or equal to 160 nm and less than or equal to 400 nm, and **in that** it is adapted to perform a conductivity measurement between two flashes of the xenon flash lamp.

2. A device according to claim 1, **characterized in that** it comprises a photo-catalyst deposit based on semiconductor material with a wide energy band on the substrate.

3. A device according to claim 2, **characterized in that** the wide energy band semiconductor material comprises at least one of a single oxide of a transition metal, a mixed oxide of a transition metal and of an alkali or alkaline-earth metal and of a transition metal sulfide, preferably doped.

4. A device according to claim 3, **characterized in that** the semiconductor material is chosen from the group comprising TiO₂, ZnO, Fe₂O₃, ZrO₂, Ta₂O₅, SrTiO₃, CaTiO₃, KTaO₃, CdS and ZnS.

5. A device according to one of claims 1 to 4, **characterized in that** it comprises an optical sensor (36) arranged at the rear face of the substrate to detect the ultraviolet rays emitted by the xenon flash lamp.

6. A device according to one of claims 1 to 5, **characterized in that** the cell comprises two complementary members (12, 13), one comprising a recess forming the conductivity measurement chamber (14) and the other forming the substrate (13), the liquid inlet and outlet of the cell being formed in the substrate-forming member (13).

7. A device according to one of claims 1 to 6, **characterized in that** it comprises a casing in two parts (10, 11) by virtue of which the cell is housed and means for assembly by clamping (29a-29d) the two parts suitable for ensuring the fluid-tightness of the conductivity measurement cell.

8. A device according to any one of claims 1 to 7, **characterized in that** the electrically insulating material of the substrate is chosen from the group comprising quartz glass, biaxially-oriented polyethylene terephthalate and silicon.

9. A device according to any one of claims 1 to 8, **characterized in that** the conductivity measurement electrode-forming electrically conductive material is chosen from the group comprising carbon, platinum, silver, gold, titanium and boron-doped diamond.

10. A device according to any one of claims 1 to 9, **characterized in that** the conductivity measurement electrode patterns take the form of two interleaved combs forming an interdigitated structure.

11. A device according to any one of claims 1 to 10, **characterized in that** it further consists of producing a thermistor (17) on the substrate by forming a pattern thereon with conductor or semiconductor material.

12. A device according to claim 11, **characterized in that** the material of the thermistor is chosen from the group comprising polysilicon, platinum and boron-doped diamond.

13. A device according to claim 11 or 12, **characterized in that** the or each conductivity measurement electrode-forming pattern, when produced on the underlying layer of thermistor-forming material, is separated therefrom by a deposit of electrically insulating material, preferably silicon dioxide (SiO₂) or silicon nitride (Si₃N₄).

14. A device according to any one of claims 1 to 13, **characterized in that** it comprises electronic components specific to the device, on the rear face of the substrate.

15. A device according to any one of claims 1 to 14, **characterized in that** the electrode patterns are formed by etching a layer of material deposited beforehand on the substrate or on an underlying layer itself deposited on that substrate.

## Patentansprüche

1. Eine Vorrichtung zum Messen des gesamten organischen Kohlenstoffs (TOC) einer Flüssigkeitsprobe, mit einer Vorrichtung zum Messen der Leitfähigkeit einer Flüssigkeit, insbesondere von ultrareinem Wasser, mit zwei Leitfähigkeitsmesselektroden (15,16), die geeignet sind, um eine Zellkonstante zu definieren, welche die Messung der Leitfähigkeit einer ultrareinen Flüssigkeit ermöglicht, wobei jede Elektrode die Form eines Musters aus elektrisch leitfähigem Material auf einem Substrat (13) aus einem elektrisch isolierenden Material bildet, mit einer Zelle mit einer Leitfähigkeitsmesskammer (14), die mit der Außenseite der Zelle über einen Flüssigkeitseinlass und -auslass (21,22), die in der Zelle vorgesehen sind, kommuniziert und die die Muster der Leitfähigkeitsmesselektroden zumindest teilweise abdeckt, und mit zumindest einem Fenster (12), das gegenüber ultravioletten Strahlen transparent ist, zum Ausführen einer Fotooxidation der Flüssigkeitsprobe, die sich in der Messkammer (14) befindet, **dadurch gekennzeichnet, dass** sie für die Fotooxidation eine Xenon-Blitzlampe aufweist, die ultraviolette Strahlen einer Wellenlänge von gleich oder mehr als 160 nm und gleich oder weniger als 400 nm emittiert, und dadurch, dass sie eingerichtet ist, um eine Leitfähigkeitsmessung zwischen zwei Blitzen der Xenon-Blitzlampe auszuführen.

2. Eine Vorrichtung gemäß Anspruch 1, **dadurch gekennzeichnet, dass** sie eine Fotokatalysatorablagerung basierend auf Halbleitermaterial mit einem breiten Energieband auf dem Substrat aufweist.

3. Eine Vorrichtung gemäß Anspruch 2, **dadurch gekennzeichnet, dass** das Halbleitermaterial mit breitem Energieband zumindest eines von einem einzelnen Oxid eines Übergangsmetalls, einem Mischoxid eines Übergangsmetalls und eines Alkali- oder Erdalkalimetalls und eines Übergangsmetallsulfids, vorzugsweise dotiert, aufweist.

4. Eine Vorrichtung gemäß Anspruch 3, **dadurch gekennzeichnet, dass** das Halbleitermaterial ausgewählt ist aus der Gruppe umfassend TiO₂, ZnO, Fe₂O₃, ZrO₂, Ta₂O₅, SrTiO₃, CaTiO₃, KTaO₃, CdS and ZnS.

5. Eine Vorrichtung gemäß einem der Ansprüche 1 bis 4, **dadurch gekennzeichnet, dass** sie einen optischen Sensor (36) aufweist, der an der Rückfläche des Substrats angeordnet ist, um die von der Xenon-Blitzlampe emittierten ultravioletten Strahlen zu erfassen.

6. Eine Vorrichtung gemäß einem der Ansprüche 1 bis 5, **dadurch gekennzeichnet, dass** die Zelle zwei komplementäre Elemente (12,13) aufweist, von denen eines eine Ausnehmung aufweist, die die Leitfähigkeitsmesskammer (14) bildet, und die andere das Substrat (13) bildet, wobei der Flüssigkeitseinlass und -auslass der Zelle in dem Substrat-Bildungselement (13) ausgebildet sind.

7. Eine Vorrichtung gemäß einem der Ansprüche 1 bis 6, **dadurch gekennzeichnet, dass** sie ein Gehäuse in zwei Teilen (10,11), mittels derer die Zelle aufgenommen ist, und Mittel zum Zusammenbau durch Klemmen (29a-29d) der zwei Teile geeignet zum Sicherstellen der Fluiddichte der Leitfähigkeitsmesszelle, aufweist.

8. Eine Vorrichtung gemäß einem der Ansprüche 1 bis 7, **dadurch gekennzeichnet, dass** das elektrisch isolierende Material des Substrats aus der Gruppe ausgewählt ist, die Quarzglas, biaxial-orientiertes Polyethylenterephthalat und Silikon aufweist.

9. Eine Vorrichtung gemäß einem der Ansprüche 1 bis 8, **dadurch gekennzeichnet, dass** das die Leitfähigkeitsmesselektrode bildende elektrisch leitfähige Material ausgewählt ist aus der Gruppe umfassend Kohlenstoff, Platin, Silber, Gold, Titan und Bor-dotierter Diamant.

10. Eine Vorrichtung gemäß einem der Ansprüche 1 bis 9, **dadurch gekennzeichnet, dass** die Leitfähigkeitsmesselektrodenmuster die Form von zwei ineinander verschachtelten Kämmen annehmen, welche eine fingerartig verbundene Struktur bilden.

11. Eine Vorrichtung gemäß einem der Ansprüche 1 bis 10, **dadurch gekennzeichnet, dass** sie ferner besteht aus dem Herstellen eines Thermistors (17) auf dem Substrat durch Ausbilden eines Musters darauf mit Leiter- oder Halbleitermaterial.

12. Eine Vorrichtung gemäß Anspruch 11, **dadurch gekennzeichnet, dass** das Material des Thermistors ausgewählt ist aus der Gruppe umfassend Polysilizium, Platin und Bordotierter Diamant.

13. Eine Vorrichtung gemäß Anspruch 11 oder 12, **dadurch gekennzeichnet, dass** das oder jedes Leitfähigkeitsmesselektroden-Bildungsmuster, wenn es auf der darunterliegenden Lage von Thermistor-Bildungsmaterial hergestellt ist oder wird, davon durch eine Ablagerung von elektrisch isolierendem Material, vorzugsweise von Siliziumdioxid (SiO₂) oder Siliziumnitrid (Si₃N₄) getrennt ist.

14. Eine Vorrichtung gemäß einem der Ansprüche 1 bis 13, **dadurch gekennzeichnet, dass** sie elektronische Komponenten, die für die Vorrichtung spezifisch sind, auf der Rückfläche des Substrats aufweist.

15. Eine Vorrichtung gemäß einem der Ansprüche 1 bis 14, **dadurch gekennzeichnet, dass** die Elektrodenmuster durch Ätzen einer Lage von Material, das zuvor auf dem Substrat abgelagert wurde, oder auf einer darunterliegenden Lage, die selbst auf dem Substrat abgelagert wurde, abgelagert wurde, ausgebildet sind oder werden.

## Revendications

1. Dispositif de mesure de la teneur en carbone organique total d'un échantillon de liquide comprenant un dispositif de mesure de conductivité d'un liquide, notamment de l'eau ultrapure, du genre comportant deux électrodes (15, 16) de mesure de conductivité propres à définir une constante de cellule permettant la mesure de la conductivité d'un liquide ultrapur, chaque électrode se présentant sous la forme d'un motif de matière électriquement conductrice sur un substrat (13) en matière électriquement isolante, une cellule ayant une chambre (14) de mesure de conductivité communiquant avec l'extérieur de la cellule par une entrée et une sortie de liquide (21, 22) ménagées dans la cellule, et recouvrant au moins partiellement les motifs des électrodes de mesure de conductivité, et au moins une fenêtre (12) transparente aux rayons ultraviolets pour effectuer une photo-oxydation de l'échantillon de liquide se trouvant dans la chambre de mesure (14), **caractérisé en ce qu'**il comporte, pour la photo-oxydation, une lampe flash au xénon émettant des rayons ultraviolets à une longueur d'onde supérieure ou égale à 160 nm et inférieure ou égale à 400 nm, et **en ce qu'**il est adapté à effectuer une mesure de conductivité entre deux impulsions de la lampe flash au xénon.

2. Dispositif selon la revendication 1, **caractérisé en ce qu'**il comporte un dépôt de photo-catalyseur à base de matière semi-conductrice à large bande d'énergie sur le substrat.

3. Dispositif selon la revendication 2, **caractérisé en ce que** la matière semi-conductrice à large bande d'énergie comprend l'un au moins d'un oxyde simple d'un métal de transition, d'un oxyde mixte d'un métal de transition et d'un métal alcalin ou alcalino-terreux et d'un sulfure de métal de transition, de préférence dopé.

4. Dispositif selon la revendication 3, **caractérisé en ce que** la matière semi-conductrice est choisie dans le groupe comprenant Ti0₂, ZnO, Fe₂O₃, ZrO₂, Ta₂O₅, SrTiO₃, CaTiO₃, KTaO₃, CdS et ZnS.

5. Dispositif selon l'une des revendications 1 à 4, **caractérisé en ce qu'**il comporte un capteur optique (36) agencé du côté du revers du substrat pour détecter les rayons ultraviolets émis par la lampe à rayons ultraviolets.

6. Dispositif selon l'une des revendications 1 à 5, **caractérisé en ce que** la cellule comporte deux éléments complémentaires (12, 13), l'un comportant un évidement formant la chambre (14) de mesure de conductivité et l'autre formant le substrat (13), les entrée et sortie de liquide de la cellule étant ménagées dans l'élément formant substrat (13).

7. Dispositif selon l'une des revendications 1 à 6, **caractérisé en ce qu'**il comporte un boîtier en deux parties (10, 11) à la faveur duquel est logée la cellule et des moyens d'assemblage par serrage (29a-29d) des deux parties propres à assurer l'étanchéité de la cellule de mesure de conductivité.

8. Dispositif selon l'une quelconque des revendications 1 à 7, **caractérisé en ce que** la matière électriquement isolante du substrat est choisie dans le groupe comprenant le verre de quartz, le polyéthylène téréphtalate bi-orienté et le silicium.

9. Dispositif selon l'une quelconque des revendications 1 à 8, **caractérisé en ce que** la matière électriquement conductrice formant électrode de mesure de conductivité est choisie dans le groupe comprenant le carbone, le platine, l'argent, l'or, le titane, et le diamant dopé au bore.

10. Dispositif selon l'une quelconque des revendications 1 à 9, **caractérisé en ce que** les motifs d'électrodes de mesure de conductivité se présentent sous la forme de deux peignes imbriqués formant une structure interdigitée.

11. Dispositif selon l'une quelconque des revendications 1 à 10, **caractérisé en ce qu'**il consiste en outre à réaliser une thermistance (17) sur le substrat en formant un motif sur celui-ci avec de la matière conductrice ou semi-conductrice.

12. Dispositif selon la revendication 11, **caractérisé en ce que** la matière de la thermistance est choisie dans le groupe comprenant le polysilicium, le platine et le diamant dopé au bore.

13. Dispositif selon la revendication 11 ou 12, **caractérisé en ce que** le ou chaque motif formant électrode de mesure de conductivité, lorsque réalisé sur la couche sous-jacente de matière formant thermistance, est séparé de celle-ci par un dépôt de matière électriquement isolante, de préférence de l'oxyde de silicium (SiO₂) ou du nitrure de silicium (Si₃N₄).

14. Dispositif selon l'une quelconque des revendications 1 à 13, **caractérisé en ce qu'**il comporte des composants électroniques propres au dispositif, sur le revers du substrat.

15. Dispositif selon l'une quelconque des revendications 1 à 14, **caractérisé en ce que** les motifs d'électrodes sont réalisés par gravure d'une couche de matière préalablement déposée sur le substrat ou sur une couche sous-jacente elle-même déposée sur ce substrat.
